# EUROPEAN PATENT APPLICATION

(11) **EP 4 803 018 A1**
(43) Date of publication of application: **09.09.2026**
(21) Application number: 26160557.0
(22) Date of filing: 25.02.2026
(51) Int. Cl.: A61B 17/72, A61B 17/80, A61B 17/84

(54) **ADJUNCTIVE PLATE FOR LONG BONE FIXATION SYSTEM**

(30) Priority: 03.03.2025 US 202563765928 P
(71) Applicant: Stryker European Operations Limited, Carrigtwohill T45H X08 (IE)
(72) Inventor: ZANDER, Nils, 24340 Eckernförde (DE)
(74) Representative: Maiwald GmbH

(57) **Abstract**

The present disclosure is directed towards an adjunctive bone plate for a nail anchored within an intramedullary canal of a bone. The plate comprises a first opening configured to receive a first bone screw anchored into the bone and fixed to the nail, and a second opening configured to receive a second bone screw anchored into the bone and fixed to the nail. The second opening is configured to receive the second bone screw such that a distance between the anchored first bone screw and anchored second bone screw is variable. The plate further comprises a third opening configured to receive a third bone screw anchored into the bone and adjacent to the nail, wherein the third opening is configured to direct the third bone screw along a support direction relative to a central longitudinal axis of the third opening.

## Description

### CROSS-REFERENCE TO RELATED APPLICATIONS

This application claims the benefit of the filing date of United States Provisional Patent Application No. 63/765,928, filed March 3, 2025, the disclosure of which is hereby incorporated herein by reference.

### BACKGROUND OF THE INVENTION

The present disclosure relates to a bone fixation system for securing a fractured bone. While discussed largely in connection with use in a proximal humerus bone, the nails, plates, and screws of the bone fixation system and the present disclosure are applicable to other types of surgical uses as well.

Fractures are often the result of traumatic injuries. It is well known in the orthopedic arts to reduce and fix a bone fracture with a nail and/or bone plate. For instance, fractures of the proximal humerus are often treated using nails and/or bone plates specifically designed to treat such bone fractures. The proximal humerus often has poor bone quality and does not support good screw purchase, particularly in the medial calcar region. To provide additional stabilization to the medial calcar region, bone plates often incorporate variable angle locking screws to effectively support the medial calcar region in order to avoid a varus malalignment of the head fragment and to maintain an adequate head-shaft angle. However, implantation of a nail and variable angle locking screws are difficult due to the precision necessary to prevent either implant from compromising the other, or further damaging the fractured bone.

Current bone fixation systems are not optimal for good screw purchase in the proximal humerus, as well as other similar bones. This often results in inadequate repair of the bone with typical nail and screw constructs. Thus, it would be desirable for a bone fixation system which provides improved precision and adjustability for the bone screws to fixate the bone plate to both the already implanted nail and bone.

### BRIEF SUMMARY OF THE INVENTION

In accordance with a first aspect of the present disclosure, a first embodiment includes an adjunctive bone plate for a nail anchored within an intramedullary canal of a bone. The plate comprises a first opening configured to receive a first bone screw anchored into the bone and fixed to the nail; a second opening configured to receive a second bone screw anchored into the bone and fixed to the nail, wherein the second opening is configured to receive the second bone screw such that a distance between the anchored first bone screw and anchored second bone screw is variable; and a third opening configured to receive a third bone screw anchored into the bone and adjacent to the nail, wherein the third opening is configured to direct the third bone screw along a support direction relative to a central longitudinal axis of the third opening extending through an anterior/posterior plane defined by the bone and a superior/inferior plane defined by the bone.

In a second embodiment of the first aspect, the support direction extends through the anterior/posterior plane 15 degrees from the central longitudinal axis. In a third embodiment of the first aspect, the support direction extends through the superior/inferior plane 15 degrees from the central longitudinal axis. In a fourth embodiment of the first aspect, the third screw is a variable angle locking screw. In a fifth embodiment of the first aspect, the first and the second screws are both intramedullary nail locking screws. In a sixth embodiment of the first aspect, the second opening has an oblong shape. In a seventh embodiment of the first aspect, the second opening is U-shaped. In an eight embodiment of the first aspect, the second opening is entirely enclosed by the plate. In a ninth embodiment of the first aspect, the second opening is adjacent a first end of the plate and the third opening is adjacent a second end of the plate opposite the first end. In a tenth embodiment of the first aspect, the second opening defines the first end of the plate.

In an eleventh embodiment of the first aspect, the plate further comprises a fourth opening adjacent the third opening, the fourth opening configured to receive a fourth bone screw anchored into the bone and adjacent to the nail and the third bone screw. In a twelfth embodiment of the first aspect, the eleventh embodiment further comprises the fourth opening is distal the third opening along a length of the plate. In a thirteenth embodiment of the first aspect, the eleventh embodiment further comprises the third opening is adjacent a first side of the plate, and the fourth opening is adjacent a second side of the plate. In fourteenth embodiment of the first aspect, the bone is a humerus, and the third bone screw is configured to be anchored in a calcar region of the humerus.

In a second aspect of the present disclosure, a first embodiment includes a system. The system comprises the adjunctive bone plate of the first embodiment of the first aspect; an intramedullary nail configured to be anchored within the bone; and a plurality of bone fixation screws configured to secure the plate to the nail and the bone.

In a third aspect of the present disclosure, a first embodiment includes a method of fixating a bone plate to a bone and a nail anchored within an intramedullary canal of the bone. The method comprises anchoring a first bone screw through a first opening of the plate through the bone and into the nail; anchoring a second bone screw through a second opening of the plate through the bone and into the nail; anchoring a third bone screw through a third opening of the plate through the bone and adjacent to the nail, wherein the third bone screw extends along a support direction relative to a central longitudinal axis of the third opening extending through a sagittal plane defined by the bone and a coronal plane defined by the bone.

In a second embodiment of the third aspect, the support direction extends through the sagittal plane 15 degrees from the central longitudinal axis. In a third embodiment of the third aspect, the support direction extends through the coronal plane 15 degrees from the central longitudinal axis. In a fourth embodiment of the third aspect, the third screw is a variable angle locking screw. In a fifth embodiment of the third aspect, the first and the second screws are both intramedullary nail locking screws. In a sixth embodiment of the third aspect, the second opening is configured to receive the second bone screw such that a distance between the anchored first bone screw and anchored second bone screw is variable.

### BRIEF DESCRIPTION OF THE DRAWINGS

A more complete appreciation of the subject matter of the present disclosure and the various advantages thereof may be realized by reference to the following detailed description which refers to the accompanying drawings, in which:
FIGS. 1A and 1B are different illustrations of various components of a bone fixation system in accordance with an embodiment of the present invention shown implanted on and within a bone;
FIG. 1C is an enlarged view of a bone plate of the bone fixation system of FIGS. 1A and 1B shown implanted on the bone;
FIG. 1D is enlarged view of alternative embodiments of a bone screw opening of the bone plate of FIG. 1C;
FIG. 2 is an illustration of various components of a bone fixation system in accordance with another embodiment of the present invention shown implanted on and within a bone;
FIG. 3 is an illustration of various components of a bone fixation system in accordance with another embodiment of the present invention shown implanted on and within a bone;
FIGS. 4A and 4B are different illustrations of various components of a bone fixation system in accordance with another embodiment of the present invention shown implanted on and within a bone; and
FIG. 5 is a perspective view of a targeting guide for use with any of the bone fixation systems of the present invention.

### DETAILED DESCRIPTION

As used herein unless stated otherwise, the term "anterior" means toward the front part of the body, and the term "posterior" means toward the back part of the body. When referring to specific directions in the following description, the terms "proximal" and "distal" are to be understood in regard to the device's orientation and position during exemplary application to human body. Thus, the term "proximal" means closer to the operator or in a direction toward the operator, and the term "distal" means more distant from the operator or in a direction away from the operator. In addition, the terms "about," "generally," and "substantially" are intended to mean that deviations from absolute are included within the scope of the term so modified.

Turning now to the figures, a bone fixation system is shown and will be discussed below. The particular examples shown permit the fixation of a bone plate to an intramedullary (IM) nail and a bone using a plurality of bone screws. However, the various examples of the bone fixation system disclosed below are not limited to just the specific designs and uses disclosed herein. For instance, while shown generally as a humeral bone fixation system, the present invention has applicability for other types of bone fixation systems and bone plates (*e.g.,* for other long bones, craniomaxillofacial, small fragments, or the like).

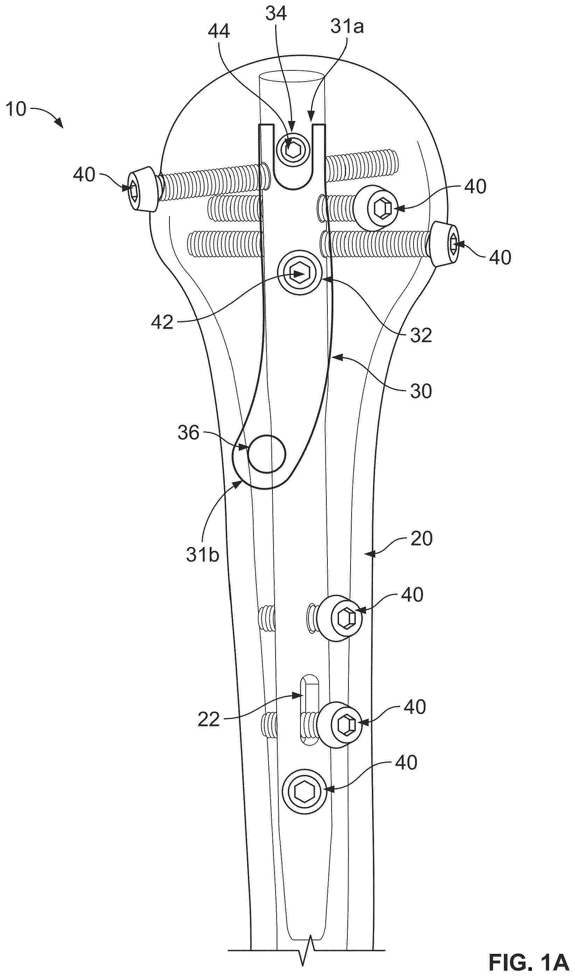

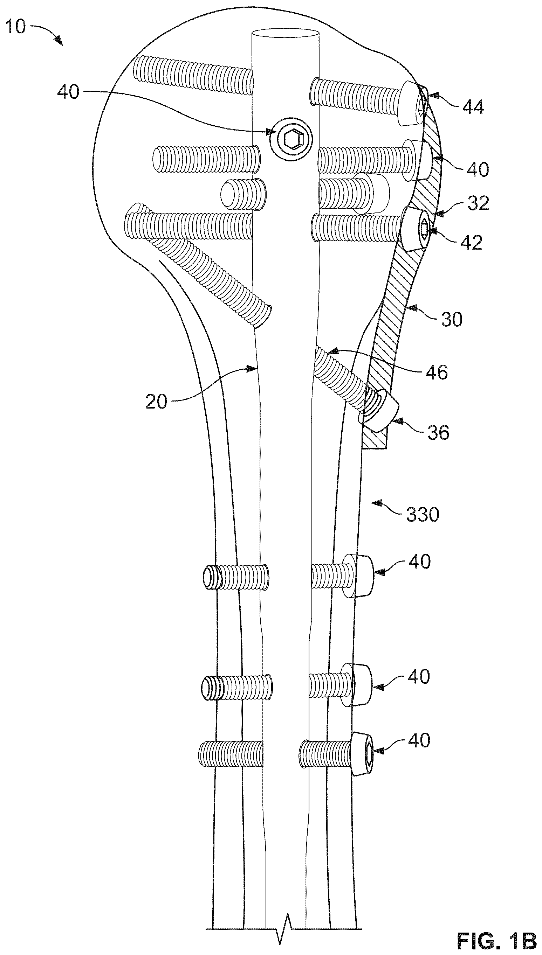

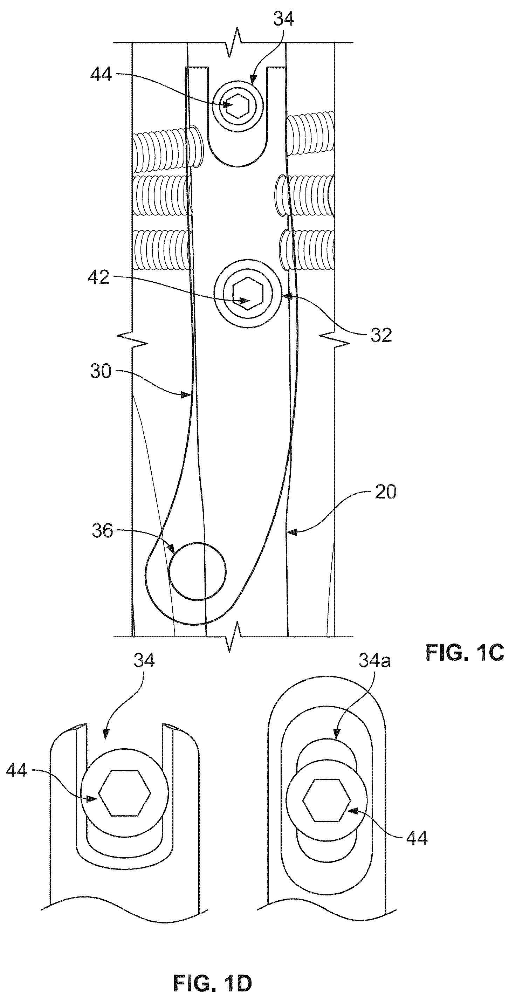

In one aspect, the present disclosure relates to a bone fixation system 10. As shown in FIGS. 1A-1D, in accordance with one embodiment of the present disclosure, bone fixation system 10 includes an intramedullary (IM) nail 20, a support or adjunctive bone plate 30, and a plurality of bone screws 40.

Nail 20 comprises a plurality of openings 22 to receive a respective bone screw 40 of the plurality of bone screws 40, as discussed further below. Each opening of the respective plurality of openings 22 may have a similar or specific configuration depending upon the type of bone fixation system 10 being utilized. Although a specific nail construct is shown in the figures it is to be understood that the present invention has applicability to various nail designs.

Plate 30 has a generally uniform shape and is curved along a portion of its length extending from a first end 31a to a second end 31b. Plate 30 comprises first, second, and third openings 32, 34, 36 each extending through a thickness of plate 30. Each of first, second, and third openings 32, 34, 36 are also configured to receive a respective bone screw 40 of the plurality of bone screws 40, as discussed further below.

First opening 32 has a substantially circular shape and is located at a substantially central position of plate 30. As discussed further below, first opening 32 is designed to receive a first bone screw 42 which would extend through the bone and into an opening 22 of nail 20 to fixate plate 30 to both the bone and the nail.

Second opening 34 has a partially oblong shape and partially defines first end 31a of plate 30. For example, as best shown in FIG. 1D, second opening 34 is U-shaped such that at least a portion of second opening 34 is not enclosed by plate 30. However, in another embodiment of second opening 34a, as best shown in FIG. 1D, second opening 34a has an oblong shape that is fully enclosed by plate 30. Additionally, second opening 34a is adjacent to first end 31a of plate 30 but does not define first end 31a of plate 30. As discussed further below, second opening 34 is designed to receive a second bone screw 44 which would extend through the bone and into an opening 22 of nail 20 to fixate plate 30 to the bone and the nail.

Third opening 36 has a substantially circular shape and is located adjacent to a second end 31b of plate 30. In the embodiment shown, at least the third opening 36 is a variable angle hole, which permits bones screws to be placed at various angles therethrough. However, in other embodiments, first and second openings 32, 34 may also be variable angle holes. For example, the variable angle holes may be holes like those disclosed in U.S. Patent No. 11,039,865, the disclosure of which is hereby incorporated by reference herein. Of course, in other embodiments, different variable angle hole designs may be employed. In other embodiments, the holes can be single axis holes or can include threading. These non-variable holes would include axes aligned to require placement of the screws in accordance with the foregoing discussion. For instance, threaded holes would have fixed angle axes aligned in the manners disclosed above. In alternative embodiments, the screw holes may include additional fixation features, such as scallops, or the like.

Additionally, in accordance with the present invention, calcar screw trajectories can reduce the relative motion of the bone along the screws. These trajectories therefore can result in a higher resistance to pull-out of screws or to bone collapse. In the prior art, screws are parallel to each other, so the bone is prone to collapsing since the mechanical resistance is in one direction. The orientation of calcar the prevents bone collapse because of the additional mechanical resistance. Additionally, the threading of the screws provides further support to the bone to prevent bone collapse.

Plate 30, and any other components or implant in accordance with the present invention, can be made of any material suitable for implanting into a human body, including but not limited to polymer materials (*e.g.,* PEEK) and metallic materials (*e.g.,* stainless steel or titanium). Plates according to the present invention can be manufactured utilizing any known process, including but not limited to additive manufacturing or the like. It is also contemplated to form certain portions of plate 30 via one process and others via another process. For instance, plate 30 can be 3D printed, with the plate openings thereafter being formed in the plate.

Any combination of components of system 10 may also be included in a single package or in individual packages which may later be brought together to create a kit. It is also contemplated that a kit may include any combination of components of system 10 along with one or more additional instruments used to prepare for and place such components in a patient. In other examples, the kits contemplated herein may be accompanied by an instruction manual on how to perform one or more of the methods of using the contents of the kit. In one embodiment, the present disclosure relates to a kit including a combination of components including at least one component of the system 10. In some embodiments, a kit may include one or more components from system 10. For example, the kit may include one or more nails 20, one or more plates 30, or one or more screws 40. Each of the one or more nails 20, plates 30, and screws 40 may be comprised of varying sizes and configurations.

In another aspect, the present disclosure relates to a method of securing a bone fracture using a bone fixation system 10. The method generally includes fixation of a bone plate 30 to an outer surface of a bone and an already implanted nail 20 via a plurality of bone screws 40. Prior to fixating bone plate 30, nail 20 is anchored into the bone via a plurality of bone screws 40 inserted through an outer surface of the bone and into openings 22 of nail 20. In other embodiments, nail is implanted into the bone and is further anchored via a plurality of bone screws 40 simultaneously with fixation of bone plate 30. In still further embodiments, nail is implanted into the bone and bone plate 30 is fixed to the bone and nail 20 prior to inserting the plurality of bone screws 40 into respective openings 22 of nail 20 that are not also received though openings of bone plate 30.

After implanting nail 20, bone plate 30 is positioned against the outer surface of the bone. Bone plate 30 may be any standard or patient specific bone plate used for securing a bone fracture. First bone screw 42 is driven through first opening 32 of bone plate 30, through the bone and into an opening 22 of nail. First bone screw 42 firmly anchors bone plate 30 to the bone and nail 20, respectively, to create a first point of fixation.

After fixating bone plate 30 to the bone and nail 20 via first bone screw 42, second bone screw 44 is driven through second opening 34 of bone plate 30, through the bone and into an opening 22 of nail 20. Second bone screw 44 firmly anchors bone plate 30 to the bone and nail 20, respectively, to create a second point of fixation in addition to the first point of fixation mentioned above. With the first and second points of fixation created, bone plate 30 will be prevented from rotation about either first or second bone screw 42, 44 and along the outer surface of the bone. Depending upon the particular anatomy or needs of the patient, a distance between anchored first and second bone screw 42, 44 will vary. For example, fixation of a bone plate 30 to a proximal humerus bone of a patient may require specific points of fixation of first and second bone screws 42, 44 to properly fixate bone plate 30 to bone and nail 20. As discussed above, second opening 34 has a substantially oblong or U-shape that defines a larger surface area than compared to that of first opening 32. This larger surface area of second opening 34 allows for second bone screw 44 to fixate bone plate 30 to the bone and nail 20 at different positions and along different trajectories depending upon the particular anatomy or needs of the patient.

After fixating bone plate 30 to the bone and nail 20 via first and second bone screws 42, 44, third bone screw 46 is driven through third opening 36 of bone plate 30 and into the bone. Third bone screw 46 is designed to provide added stability for bone fixation system 10 and desired portions of the bone fracture of the patient. Additionally, third bone screw 46 is a variable angle locking screw configured to be inserted through third opening 36 of bone plate 30 along various trajectories depending upon the particular anatomy and needs of the patient.

In the depicted embodiment, third bone screw 46 is inserted into a medial calcar region of a proximal humerus fracture, and specifically within an anterior portion of the proximal humerus. Third bone screw 46 is inserted through third opening 36 and into the bone along a central longitudinal axis extending though third opening 36. However, depending upon the particular anatomy and needs of the patient, third bone screw 46 may be inserted into the bone along desired or alternative trajectories relative to the central longitudinal axis. For example, third bone screw 46 may be inserted at least 15 degrees greater or less than the central longitudinal axis through an anterior/posterior plane defined by the proximal humerus. Additionally, or alternatively, third bone screw 46 may be inserted at least 15 degrees greater or less than the central longitudinal axis through a superior/posterior plane defined by the proximal humerus. Determination of a desired or alternative trajectory of third bone screw 46 may be decided preoperatively and/or intraoperatively with the use of imaging technology (*i.e.,* X-rays) on the anatomy of the patient. Further, it is to be understood that this method may differ based on surgeon preference, location of the fracture lines, or the needs of the patient.

One benefit of third bone screw 46 being designed to be selectively inserted into the bone within both the anterior/posterior and superior/posterior planes is to increase the accuracy of third bone screw 46 placement within the medial calcar region of the anatomy of the patient. For example, selective placement along the desired trajectory prevents third bone screw 46 from contacting the axillary nerve, also known as the circumflex nerve, of the shoulder of the patient. Additionally, insertion of third bone screw 46 designed to avoid nerves and other anatomy of the patient determined to be undesirable for implantation when used with bone fixation system 10.

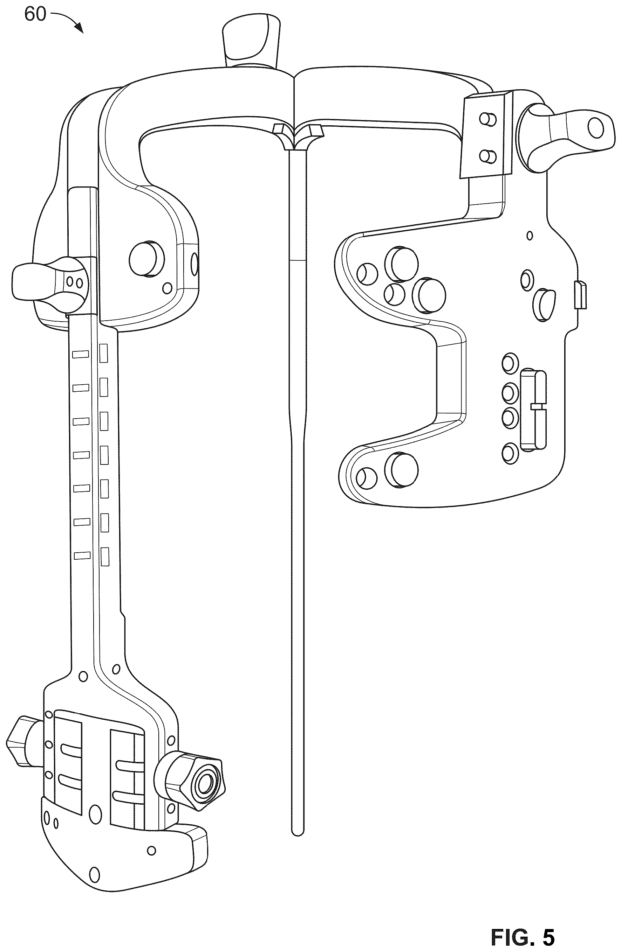

The foregoing methodology may involve the use of standard tools, such as screw drivers and drills to prepare the bone and place the screws. Guides, such as drill and screw placement guides, can also be utilized. For example, a targeting guide 60 is shown in FIG. 5. This is particularly true in connection with embodiment plates having variable angle screw holes. The use of guides can dictate the placement of the screws according to the orientations described above. For instance, such guides may force the screws into the convergence, standard, convergence, and calcar screw trajectories noted above.

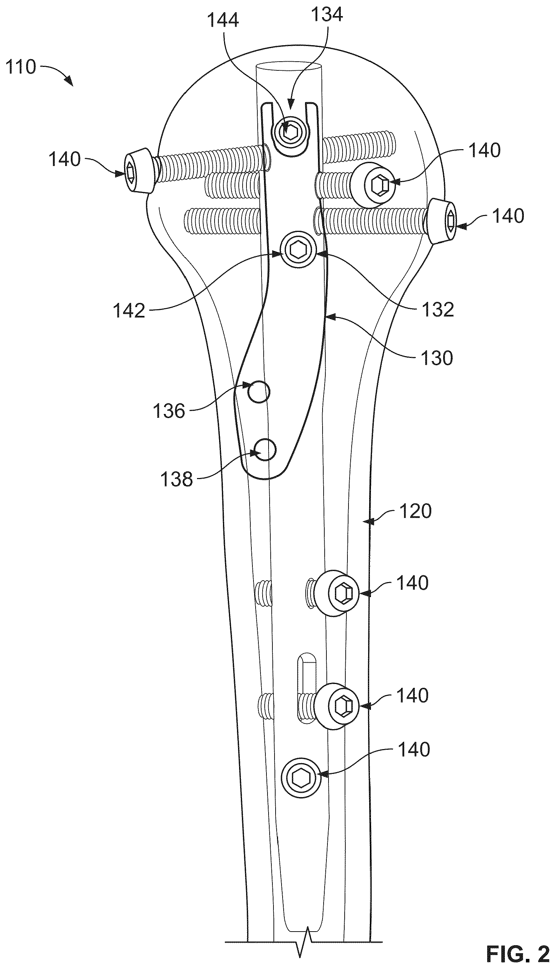

Another embodiment of a bone fixation system 10 is shown in FIG. 2 with like reference numerals being utilized in connection with similar components to that of bone fixation system 10, but within the 100-series of numbers. For instance, bone fixation system 110 includes an intramedullary (IM) nail 120, a support or adjunctive bone plate 130, and a plurality of bone screws 140. In fact, the below discussion is focused on the only component that significantly differs from that of bone fixation system 10 - plate 130.

Plate 130 has a generally uniform shape and is curved along a portion of its length extending from a first end 131a to a second end 131b. Plate 130 comprises first, second, third, and fourth openings 132, 134, 136, 138 each extending through a thickness of plate 130. Each of first, second, third, and fourth openings 132, 134, 136, 138 also are configured to receive a respective bone screw of the plurality of bone screws 140, as discussed further below.

First opening 132 has a substantially circular shape and is located at a substantially central position of plate 130. Second opening 134 has a partially oblong shape and partially defines first end 131a of plate 130. Second opening 134 may be alternatively designed in manner similar to that of second opening 34a of system 10, as mentioned above.

Third opening 136 has a substantially circular shape and is located proximal to fourth opening 138 of plate 130. Fourth opening 138 has a substantially circular shape and is located adjacent to a second end 131b of plate 130 and distal third opening 136 along a longitudinal length of plate 130.

A method of using bone fixation system 110 is substantially similar to the method of using bone fixation system 10, as mentioned above. In fact, the method of using bone fixation system 110 differs only in that a fourth bone screw (not shown) is inserted through fourth opening 138 of bone plate 130. Similar to that of third bone screw (not shown), fourth bone screw is inserted into a medial calcar region of a proximal humerus fracture, and specifically within an anterior portion of the proximal humerus. Fourth bone screw is inserted through fourth opening 138 and into the bone along a central longitudinal axis extending though fourth opening 138. However, depending upon the particular anatomy and needs of the patient, fourth bone screw may be inserted into the bone along desired or alternative trajectories relative to the central longitudinal axis. For example, fourth bone screw may be inserted at least 15 degrees greater or less than the central longitudinal axis through an anterior/posterior plane defined by the proximal humerus.

Additionally, or alternatively, fourth bone screw may be inserted at least 15 degrees greater or less than the central longitudinal axis through a superior/posterior plane defined by the proximal humerus. Determination of a desired or alternative trajectory of fourth bone screw may be decided preoperatively and/or intraoperatively with the use of imaging technology (*i.e.,* X-rays) on the anatomy of the patient. Thus, fourth bone screw provides additional support of bone plate 130 to the bone when used with third bone screw. Alternatively, third bone screw may not be used with bone fixation system 10, and only fourth bone screw may be inserted through bone plate 130 and into the bone.

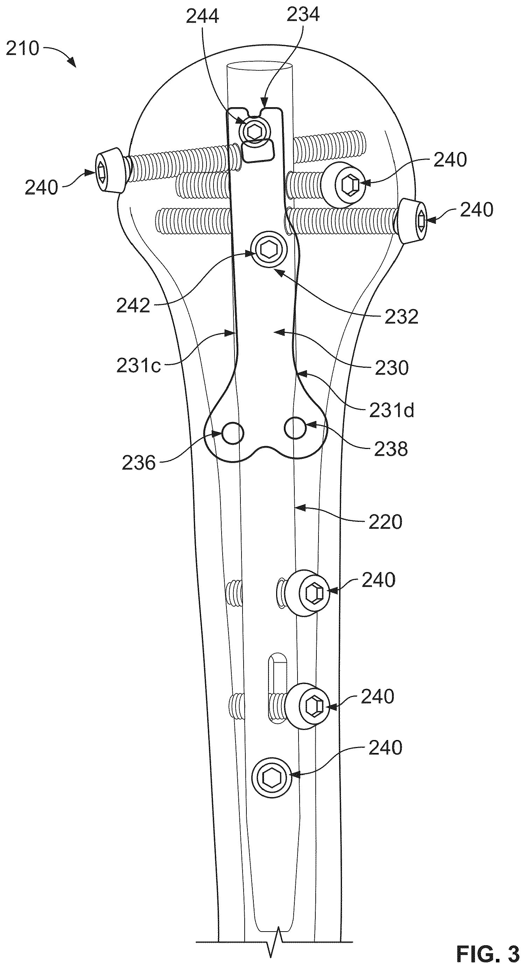

Another embodiment of a bone fixation system 210 is shown in FIG. 3 with like reference numerals being utilized in connection with similar components to that of bone fixation system 10, 110, but within the 200-series of numbers. For instance, bone fixation system 210 includes an intramedullary (IM) nail 220, a support or adjunctive bone plate 230, and a plurality of bone screws 240. In fact, the below discussion is focused on the only component that significantly differs from that of bone fixation system 10, 110 - plate 230.

Plate 230 has a generally uniform shape extending from a first end 231a to a portion of plate 230 adjacent to a second end 231b. Second end 231b is substantially triangular in shape and has a width greater than first end 231a of plate 230.

Plate 230 comprises first, second, third, and fourth openings 232, 234, 236, 238 each extending through a thickness of plate 230. Each of first, second, third, and fourth openings 232, 234, 236, 238 also are configured to receive a respective bone screw of the plurality of bone screws 240, as discussed further below.

First opening 232 has a substantially circular shape and is located at a substantially central position of plate 230. Second opening 234 has a partially oblong shape and partially defines first end 231a of plate 230. Second opening 234 may be alternatively designed in manner similar to that of second opening 34a of system 10, as discussed above.

Third and fourth openings 236, 238 each have a substantially circular shape and are each located adjacent to second end 231b of plate 230. Additionally, third opening 236 is positioned on a first side 231c of plate 230, and fourth opening 238 is positioned on second side 231b of plate 230. First and second sides 231c, 231d of plate 230 opposite one another and each are separated by central longitudinal axis extending through a length of plate 230.

A method of using bone fixation system 210 is substantially similar to the method of using bone fixation systems 10, 110, as mentioned above. In fact, the method of using bone fixation system 210 differs only in that a fourth bone screw (not shown) is inserted through fourth opening 238 of bone plate 230. Similar to that of third bone screw (not shown), fourth bone screw is inserted into a medial calcar region of a proximal humerus fracture, and specifically within a posterior portion of the proximal humerus. Fourth bone screw is inserted through fourth opening 238 and into the bone along a central longitudinal axis extending though fourth opening 238. However, depending upon the particular anatomy and needs of the patient, fourth bone screw may be inserted into the bone along desired or alternative trajectories relative to the central longitudinal axis. For example, fourth bone screw may be inserted at least 15 degrees greater or less than the central longitudinal axis through an anterior/posterior plane defined by the proximal humerus.

Additionally, or alternatively, fourth bone screw may be inserted at least 15 degrees greater or less than the central longitudinal axis through a superior/posterior plane defined by the proximal humerus. Determination of a desired or alternative trajectory of fourth bone screw may be decided preoperatively and/or intraoperatively with the use of imaging technology (*i.e.,* X-rays) on the anatomy of the patient. Thus, fourth bone screw provides additional support of bone plate 230 to the bone when used with third bone screw. Alternatively, third bone screw may not be used with bone fixation system 10, and only fourth bone screw may be inserted through bone plate 230 and into the bone.

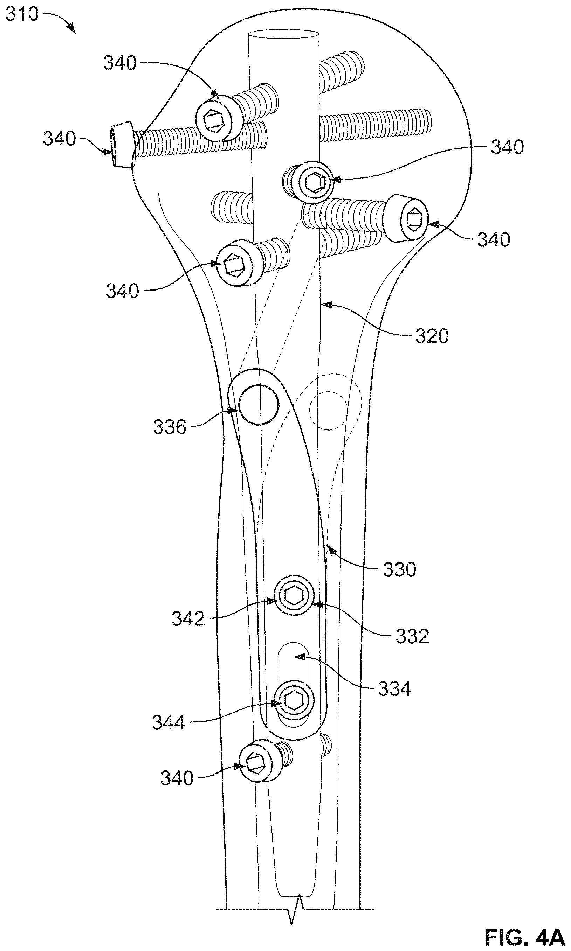

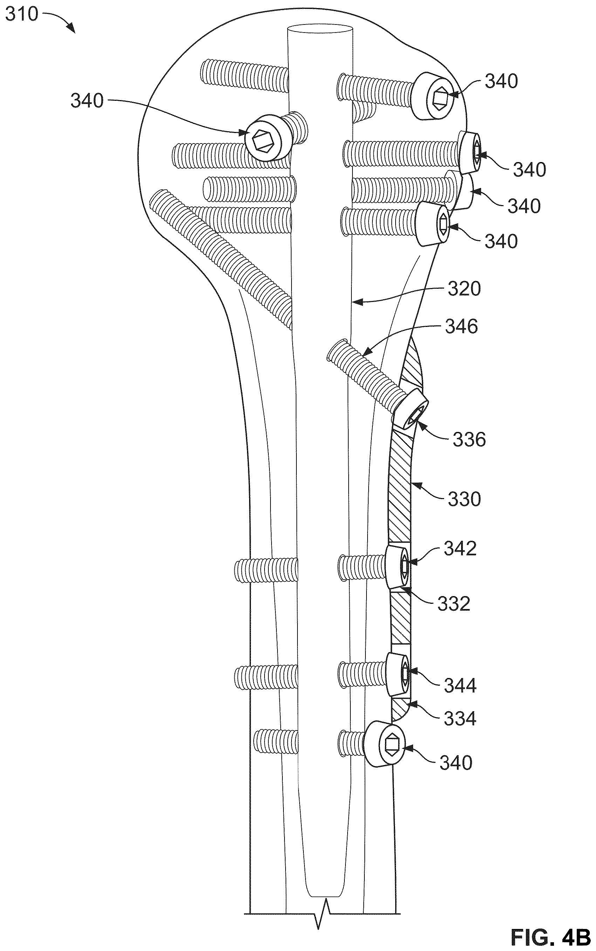

Another embodiment of a bone fixation system 310 is shown in FIGS. 4A-4B with like reference numerals being utilized in connection with similar components to that of bone fixation system 10, 110, 210, but within the 300-series of numbers. For instance, bone fixation system 310 includes an intramedullary (IM) nail 320, a support or adjunctive bone plate 330, and a plurality of bone screws 340.

A method of using bone fixation system 310 is substantially similar to the method of using bone fixation systems 10, 110, 210, as mentioned above, but the positioning and use of bone plate 330 differs from the use of the bone plates in the other bone fixations systems. For example, when compared to bone plate 30 of bone fixation system 10, bone plate 330 is positioned along a distal portion of the bone and is configured to be fixated to a distal portion of nail 320 within bone fixation system 310. Bone plate 330 is also configured for use within bone fixation system 310 in an opposite orientation of that bone plate 30, as a first end 331a is positioned along a proximal portion of the bone and second end 331b is positioned along a distal portion of the bone. As such, a third opening 336 is proximal both of the first and second openings 332, 334 of bone plate 330 when positioned against the bone. However, third opening 336 is still similarly designed to receive a third bone screw 346 inserted into the medial calcar region of the bone, and first and second openings 332, 334 are similarly designed to receive respective first and second bone screws 342, 344 inserted through the bone and into respective opening 322 of the nail 320.

It is to be understood that the disclosure set forth herein includes any possible combinations of the particular features set forth above, whether specifically disclosed herein or not. For example, where a particular feature is disclosed in the context of a particular aspect, arrangement, configuration, or arrangement, that feature can also be used, to the extent possible, in combination with and/or in the context of other particular aspects, arrangements, configurations, and arrangements of the technology, and in the technology generally.

Furthermore, although the technology herein has been described with reference to particular features, it is to be understood that these features are merely illustrative of the principles and applications of the present technology. It is therefore to be understood that numerous modifications, including changes in the sizes of the various features described herein, may be made to the illustrative arrangements and that other arrangements may be devised without departing from the spirit and scope of the present technology. In this regard, the present technology encompasses numerous additional features in addition to those specific features set forth in the claims below. Moreover, the foregoing disclosure should be taken by way of illustration rather than by way of limitation as the present technology is defined by the claims set forth below.

## Claims

1. An adjunctive bone plate for a nail anchored within an intramedullary canal of a bone, the plate comprising:
a first opening configured to receive a first bone screw anchored into the bone and fixed to the nail;
a second opening configured to receive a second bone screw anchored into the bone and fixed to the nail, wherein the second opening is configured to receive the second bone screw such that a distance between the anchored first bone screw and anchored second bone screw is variable; and
a third opening configured to receive a third bone screw anchored into the bone and adjacent to the nail, wherein the third opening is configured to direct the third bone screw along a support direction relative to a central longitudinal axis of the third opening extending through an anterior/posterior plane defined by the bone and a superior/inferior plane defined by the bone.

2. The plate of claim 1, wherein the support direction extends through the anterior/posterior plane 15 degrees from the central longitudinal axis.

3. The plate of claims 1 or 2, wherein the support direction extends through the superior/inferior plane 15 degrees from the central longitudinal axis.

4. The plate of any one of claims 1-3, wherein the third screw is a variable angle locking screw.

5. The plate of any one of claims 1-4, wherein the first and the second screws are both intramedullary nail locking screws.

6. The plate of any one of claims 1-5, wherein the second opening has an oblong shape.

7. The plate of any one of claims claim 1-5, wherein the second opening is U-shaped.

8. The plate of any one of claims 1-6, wherein the second opening is entirely enclosed by the plate.

9. The plate of any one of claims 1-8, wherein the second opening is adjacent a first end of the plate and the third opening is adjacent a second end of the plate opposite the first end.

10. The plate of any one of claims 1-7 and 9, wherein the second opening defines the first end of the plate.

11. The plate of any one of claims 1-10, further comprising a fourth opening adjacent the third opening, the fourth opening configured to receive a fourth bone screw anchored into the bone and adjacent to the nail and the third bone screw.

12. The plate of claim 11, wherein the fourth opening is distal the third opening along a length of the plate.

13. The plate of claims 11 or 12, wherein the third opening is adjacent a first side of the plate, and the fourth opening is adjacent a second side of the plate.

14. The plate of any one of claims 1-13, wherein the bone is a humerus, and the third bone screw is configured to be anchored in a calcar region of the humerus.

15. A system, comprising:
the plate of any one of claims 1-14;
an intramedullary nail configured to be anchored within the bone; and
a plurality of bone fixation screws configured to secure the plate to the nail and the bone.
